(19) Europäisches Patentamt
European Patent Office
Office européen des brevets

(11) Veröffentlichungsnummer: **0 108 323**
**A1**

# (12) EUROPÄISCHE PATENTANMELDUNG

(21) Anmeldenummer: 83110597.8

(22) Anmeldetag: 24.10.83

(51) Int. Cl.³: C 07 C 85/24

(30) Priorität: 30.10.82 DE 3240286

(43) Veröffentlichungstag der Anmeldung: 16.05.84 Patentblatt 84/20

(84) Benannte Vertragsstaaten: BE DE FR GB IT NL

(71) Anmelder: BASF Aktiengesellschaft
Carl-Bosch-Strasse 38
D-6700 Ludwigshafen(DE)

(72) Erfinder: Goetz, Norbert, Dr.
Schoefferstrasse 25
D-6520 Worms 1(DE)

(72) Erfinder: Toussaint, Herbert, Dr.
Knietschstrasse 11
D-6710 Frankenthal(DE)

(72) Erfinder: Hupfer, Leopold, Dr.
Waltershoehe 3
D-6701 Freidelsheim(DE)

(72) Erfinder: Reiss, Wolfgang, Dr.
Bannwasserstrasse 70
D-6700 Ludwigshafen(DE)

## (54) Verfahren zur Herstellung von N-alkylsubstituierten Cyclohexylaminen.

(57) Verfahren zur Herstellung von cycloaliphatischen Aminen durch Hydrierung von aromatischen Aminen in Gegenwart eines Hydrierkatalysators, wobei man einen Trägerkatalysator verwendet, der als Träger Aluminiumoxid oder eine Aluminiumoxid enthaltende Verbindung, Palladium und eine basische Verbindung und/oder ein Element der Gruppe Ib, IIb, VIIb, Eisen, Kobalt oder Nickel enthält, dadurch gekennzeichnet, daß man als aromatische Amine N-Alkyl- bzw. N-Arylaniline einsetzt.

EP 0 108 323 A1

Verfahren zur Herstellung von N-alkylsubstituierten Cyclohexylaminen

Die vorliegende Erfindung betrifft ein neues Verfahren zur Herstellung von N-Mono- bzw. N,N-Dialkylcyclohexylaminen durch Kernhydrierung entsprechend substituierter aromatischer Amine in Gegenwart von Palladiumkatalysatoren.

Zur Synthese von N-alkylsubstituierten Cyclohexylaminen werden häufig Cyclohexanderivate verwendet, deren funktionelle Gruppen dann entsprechend modifiziert werden, z.B. reduktive Aminierung von Cyclohexanonen mit primären und sekundären Aminen (siehe u.a. DE-OS 2 118 283, US-PS 3 873 621) oder Alkylierung N-unsubstituierter Cyclohexylamine (siehe u.a. DE-OS 2 324 855).

Die Verfahrensweise aromatische Amine durch Kernhydrierung in Gegenwart von auf Trägern befindlichen Edelmetallkatalysatoren in die entsprechenden Cyclohexanverbindungen zu überführen, wie dies bei der Umwandlung N-unsubstituierter Aniline in primäre Cyclohexylamine vielfach beschrieben ist, wird bei N-alkyl- bzw. N-arylsubstituierten Anilinen sehr viel seltener angewendet.

Bei den bekannten Verfahren wird dabei der Einsatz von Nickel- bzw. Rutheniumkatalysatoren vorgeschlagen (US-PS 3 376 341 bzw. J.Org.Chem.27, 3568 (1962); Chemtech. 1980, 444; DE-AS 1 593 289; JA-OS 148 043/77).

Palladiumkatalysatoren mit deren Hilfe N-unsubstituierte Aniline mit gutem Erfolg in die entsprechenden Cyclohexylamine überführt werden können (EP-OS 53 818), sind hier ohne Bedeutung, denn nach gängiger Anschauung besitzen nämlich Rutheniumkatalysatoren gegenüber Palladiumkatalysatoren den Vorteil einer größeren Aktivität und einer Verminderung der Nebenproduktbildung, insbesondere beim Einsatz von primären und sekundären Aminen (P.N. Rylander "Catalytic Hydrogenation over Platinum Metals" S. 355f, Academic Press, New York, London (1967); Ann.N.Y.Acad.Sci. 214, 233 (1973)).

Es wurde nun gefunden, daß man cycloaliphatische Amine durch Hydrierung von aromatischen Aminen in Gegenwart eines Hydrierkatalysators, wobei man einen Trägerkatalysator verwendet, der als Träger Aluminiumoxid oder eine Aluminiumoxid enthaltende Verbindung, Palladium und eine basische Verbindung und/oder ein Element der Gruppe Ib, IIb, VIIb, Eisen, Kobalt oder Nickel enthält, in guter Ausbeute und Reinheit erhält, wenn man als aromatische Amine N-Alkyl- bzw. N-Arylaniline einsetzt.

Kg/HB

Die Hydrierung von Anilinen mittels des erfindungsgemäßen Verfahrens führt zu Cyclohexylaminen der allgemeinen Formel I

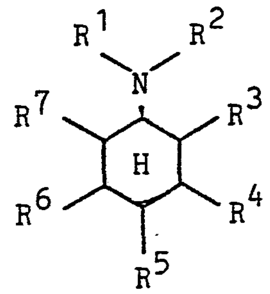

(I),

in der die beiden Reste $R^1$ und $R^2$ jeweils eine Alkylgruppe mit 1 bis 10 Kohlenstoffatomen, eine Cycloalkylgruppe mit 5 bis 15 Kohlenstoffatomen oder eine Alkylcycloalkylgruppe mit 7 bis 15 Kohlenstoffatomen bedeuten, oder einer der beiden Reste $R^1$ und $R^2$ jeweils für ein Wasserstoffatom steht, während der andere die vorgenannte Bedeutung hat und die Reste $R^3$ bis $R^7$ gleich oder verschieden sein und jeweils ein Wasserstoffatom, eine Alkylgruppe mit 1 bis 10 Kohlenstoffatomen, eine Cycloalkylgruppe mit 5 bis 15 Kohlenstoffatomen oder eine Alkylcycloalkylgruppe mit 7 bis 15 Kohlenstoffatomen sein können.

Die erfindungsgemäß zu hydrierenden Aniline lassen sich durch die allgemeine Formel II

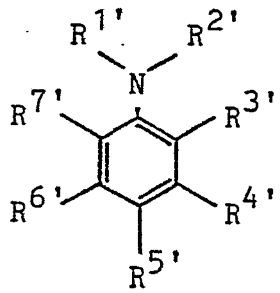

(II)

beschreiben, in der die Reste $R^{1'}$ bis $R^{7'}$ jeweils die vorgenannte Bedeutung der Reste $R^1$ bis $R^7$ besitzen und zusätzlich noch eine Arylgruppe mit 6 bis 15 Kohlenstoffatomen oder eine Aralkyl- oder Alkylarylgruppe mit 7 bis 15 Kohlenstoffatomen sein können.

Beispielsweise können folgende N-Alkyl- bzw. N-Arylaniline verwendet werden: N-Monomethylanilin, N,N-Dimethylanilin, N-Monoethylanilin, N,N-Diethylanilin, N-Isopropylanilin, N,N-Dibutylanilin, N-Cyclohexylanilin, N,N-Dimethyl-o-toluidin, N,N-Dimethyl-p-toluidin, N,N-Dimethyl-2-ethyl--anilin, N,N-Dimethyl-2-propylanilin, N,N-Dimethyl-2,6-dimethylanilin, N,N-Dimethyl-3-tert.-butylanilin, N,N-Dimethyl-3-phenyl-anilin, N-(2--Ethylhexyl)-2,6-dimethylanilin, Diphenylamin, N-Isopropyl-2,6-dimethylanilin, N-Ethyl-2-methyl-6-tert.-butylanilin, N-Ethyl-2,6-diisopropylanilin.

Das erfindungsgemäße Verfahren läßt sich kontinuierlich oder diskontinuierlich durchführen. In allen Fällen kann der Katalysator in einem Fest- oder Wirbelbett angeordnet sein oder als Suspension vorliegen. Bei kontinuierlicher Arbeitsweise, bei der ein fest angeordneter Katalysator bevorzugt ist, wird das Ausgangsprodukt zusammen mit Wasserstoff - und gegebenenfalls Ammoniak - über den Katalysator geleitet. Im diskontinuierlichen Betrieb führt man so lange Wasserstoff zu, bis das eingesetzte aromatische Amin vollständig umgesetzt ist.

Die Reaktionstemperatur liegt bei 100 bis 300°C, vorzugsweise 150 bis 250°C. Der Druck soll wenigstens 100 bar, vorzugsweise aber 150 bis 300 bar betragen.

Die Umsetzung kann mit oder ohne Lösungsmittel durchgeführt werden. Geeignete Lösungsmittel sind solche, die unter den gewählten Reaktionsbedingungen indifferent sind, beispielsweise Methanol, Ethanol, n-Butanol, Tetrahydrofuran, Dioxan, Cyclohexylmethylether, Methylglykol, Ethylglykol, 1,2-Dimethoxyethan, N-Methylpiperidin, N-Ethylpiperidin, N-Methylmorpholin, N-Methylpyrrolidin, Cyclohexan.

In vielen Fällen ist das Zielprodukt selbst das günstigste Lösungsmittel; man führt einen Teilstrom zurück, wenn eine entsprechende Verdünnung gewünscht wird.

Der Katalysator enthält Palladium auf einem Träger aus Aluminiumoxid oder einem seiner Umsetzungsprodukte. Weiterhin sind im Katalysator enthalten: Elemente bzw. Verbindungen von Elementen der Gruppen Ib, IIb und VIIb des Periodensystems sowie Eisen, Kobalt und Nickel und/oder basische Stoffe, d.h. Verbindungen der ersten bis dritten Hauptgruppe des Periodensystems. Diese basischen Stoffe bilden vorzugsweise einen Bestandteil des Trägers. Natürlich können die Zusätze allein oder im Gemisch miteinander neben dem Edelmetall vorliegen. Der Träger besteht aus Aluminiumoxid oder einem unter Mitwirkung von Aluminiumoxid aufgebauten Stoff, wie Aluminiumsilikat oder Spinelle des Aluminiums. Die Zusätze, die dem Katalysator seine Eigenschaften verleihen, können sich an der Oberfläche des Katalysators befinden oder zusammen mit dem Aluminiumoxid usw. in Form einer Mischung den Träger darstellen oder mit dem ursprünglichen Träger durch nachträgliches Tempern ein gemeinsames Kristallgitter bilden (beispielsweise bei Aluminiumoxidträgern Bildung von typischen Spinellgittern mit bestimmten Metallen). Das bedeutet, daß der Träger Aktivität und auch Lebensdauer des Katalysators günstig beeinflussen kann.

Als Zusätze kommen in Frage:

a) Basische Zusätze, wie Oxide, Hydroxide oder Carbonate der Alkalimetalle, vorzugsweise des Lithiums und Natriums, der Erdalkalimetalle, vorzugsweise des Magnesiums und Calciums und der Seltenerdmetalle, vorzugsweise des Cers und Praseodyms (Neodyms).

b) Weitere Metalle, wie Eisen, Nickel, Kobalt, Mangan, Zink, Cadmium und Silber.

Die Zusätze können gemeinsam mit dem Palladium durch Tränken des Trägermaterials mit Lösungen von z.B. Nitraten, Chloriden, Formiaten oder Oxalaten aufgebracht werden. Durch anschließende thermische Behandlung, die gewöhnlich zwischen 400 und 600°C durchgeführt wird, erfolgt Überführung in die Oxidform. Soll bei Aluminiumoxidträgern mit geeigneten Metallen (Mg, Zn, Co, Mn, Li) Spinellbildung erzielt werden, muß nach der Tränkung auf eine Temperatur von 900 bis 1300°C erhitzt werden (siehe Ullmanns Encyklopädie der techn. Chemie, 3. Auflage (1955), Band 6, S. 242 bis 244; Gmelin, System-Nr. 35, Al, Tl, 1934-1935, S. 26 bis 28) und anschließend in der üblichen Weise das Edelmetall aufgebracht werden.

Manche Zusätze, wie beispielsweise Calciumoxid oder Magnesiumoxid lassen sich mit einem Träger wie Aluminiumoxid mischen und bilden dann nach gemeinsamem Tempern bei 400 bis 600°C einen neuen Träger, auf dem das Palladium niedergeschlagen werden kann.

Der Palladiumgehalt des Katalysators, bezogen auf das Trägermaterial, liegt gewöhnlich zwischen 0,05 bis 15 Gew.%.

Das Gewichtsverhältnis der Zusätze zum Palladium kann unterschiedlich sein; es kann z.B. zwischen 10.000:1 bis 1:50, bevorzugt bei 100:1 bis 1:50 liegen. Man verwendet den Katalysator beispielsweise in Form von Strängen mit einem Durchmesser von 3 mm und einer Länge von 10 mm oder als Pulver, je nach dem vorgesehenen Anwendungszweck.

Herstellmöglichkeiten für den Katalysator

a) Auf strang- oder pulverförmiges $\gamma$-Aluminiumoxid wird das Edelmetall zusammen mit dem Zusatz (Mangan, Zink, Silber, Seltenerdmetalle u.a.)

in den gewünschten Mengen durch Tränken mit beispielsweise deren Nitratlösungen und anschließendes Eindampfen bis zur Trockene aufgebracht. Danach wird 6 Stunden bei 550°C getempert und im Wasserstoffstrom bei 300°C reduziert.

b) Auf strang- oder pulverförmiges γ-Aluminiumoxid wird zunächst die gewünschte Menge des Zusatzes (Mangan, Zink, Kobalt, Magnesium, Lithium u.a.) durch Tränken mit entsprechenden wäßrigen Nitrat- oder Formiatlösungen aufgebracht und bei 150°C getrocknet. Der derart vorbehandelte Träger wird nun entweder 6 Stunden lang auf 550°C erhitzt oder, wenn Spinellbildung erreicht werden soll, 6 Stunden lang bei 1050°C geglüht. Nun wird der Träger mit wäßriger Edelmetallnitratlösung getränkt und durch 7-stündiges Erhitzen bei 300°C im Wasserstoffstrom reduziert. Wurde zum Tränken eine Lösung eines Chlorids genommen, wird mit alkalischer Formalinlösung reduziert.

c) γ-Aluminiumoxid und ein basisches Oxid (MgO, CaO) werden in dem gewünschten Mengenverhältnis intensiv miteinander vermischt. Diese Mischung wird 6 Stunden lang auf 450°C erhitzt, anschließend mit Edelmetallnitratlösung getränkt und 7 Stunden mit Wasserstoff bei 300°C reduziert. Der Katalysator wird mit einer 5%igen (Gew.%) wäßrigen Hydrazinhydratlösung nachreduziert und dann bei 120°C getrocknet.

Nach dem erfindungsgemäßen Verfahren werden die gewünschten Cyclohexylamine bei vollständigem Umsatz und hoher Ausbeute frei von Neben- und Spaltprodukten (teilhydrierte oder entalkylierte Produkte bzw. tertiäre Amine beim Einsatz von N-Monoalkyl- bzw. N-Monoarylanilinen) gewonnen.

Speziell bei sterisch gehinderten Anilinen als Ausgangsstoffen (z.B. N-Isopropyl-2,6-dimethylanilin, N-(2-Ethylhexyl)-2,6-dimethylanilin, N-Ethyl-2-methyl-6-tert.-butylanilin) deren Kernhydrierung üblicherweise Schwierigkeiten bereitet, liefert das erfindungsgemäße Verfahren gute Ergebnisse.

Ein weiterer Vorteil des erfindungsgemäßen Verfahrens liegt darin, daß die Palladiumkatalysatoren im Vergleich zu Ruthenium- und Rhodiumkatalysatoren vielseitiger anwendbar sind und damit den Aufbau von Hydrieranlagen ermöglichen, die für verschiedene Zwecke geeignet sind. Vorteilhaft ist dabei auch die lange Lebensdauer der verwendeten Palladiumkatalysatoren; so ist beispielsweise nach einer Dauerbeanspruchung von über 1200 Betriebsstunden kein Aktivitätsabfall zu verzeichnen.

N-alkylsubstiuierte Cyclohexylamine finden als Komponenten für Vulkanisationsbeschleuniger, optische Aufheller, Katalysatoren für Polyurethane etc. Verwendung (Ullmanns Encyklopädie der techn. Chemie, 4. Auflage (1974), Band 7, S. 379).

Die in den nachfolgenden Beispielen angegebenen Siedepunkte beziehen sich, sofern nicht anders angegeben, auf atmosphärischen Druck, sonst auf verminderten Druck in mbar.

<u>Beispiel 1</u>

N,N-Dimethylcyclohexylamin
In ein druckfestes zylindrisches Rohr mit einem Volumen von 1 l als Reaktor wurde ein Katalysator in Form von Strängen (3 mm Durchmesser, 10 mm Länge), der 0,5 Gew.% Palladium auf Magnesium-Aluminiumspinell enthielt, eingefüllt und auf 180°C erhitzt. Bei dieser Temperatur wurden über den Katalysator bei einem Druck von 250 bar stündlich 150 g N,N-Dimethylanilin geführt. Gleichzeitig wurden 100 Nl Wasserstoff/Stunde durch das Reaktionsrohr hindurchgeleitet. Das abfließende Produkt wurde unter Druck abgekühlt und dann entspannt. Es fielen hierbei stündlich ca. 157,5 g N,N-Dimethylcyclohexylamin an. Man destillierte und erhielt 154 g reines N,N-Dimethylcyclohexylamin, Kp = 164 bis 165°C, entsprechend einer Ausbeute von 98 % der Theorie.

<u>Beispiel 2</u>

N-Ethylcyclohexylamin
Man verfuhr analog Beispiel 1, verwendete aber einen Katalysator, der 1,0 Gew.% Palladium auf Kobalt-Aluminiumspinell enthielt. Ausgehend von N-Ethylanilin erhielt man als Reaktionsprodukt N-Ethylcyclohexylamin (Kp = 165 bis 166°C) in einer Ausbeute von 96 % der Theorie.

<u>Beispiel 3</u>

N-Isopropylcyclohexylamin
Man verfuhr analog Beispiel 1, verwendete aber einen Katalysator, der 1,0 Gew.% Palladium auf Lithium-Aluminiumspinell enthielt. Ausgehend von N-Isopropylanilin wurde als Reaktionsprodukt N-Isopropylcyclohexylamin (Kp = 63 bis 65°C/16 mbar) in einer Ausbeute von 96 % der Theorie erhalten.

Beispiel 4

N-Butylcyclohexylamin

Man verfuhr analog Beispiel 1, arbeitete aber bei 210°C und verwendete einen Katalysator, der 2,5 Gew.% Palladium auf einer Mischung aus 19,4 Gew.% Calciumoxid und 80,6 Gew.% Aluminiumoxid enthält. Ausgehend von N-Butylanilin wurde als Reaktionsprodukt N-Butylcyclohexylamin (Kp = 207°C) in einer Ausbeute von 98 % der Theorie erhalten.

Beispiel 5

Dicyclohexylamin

In einem 300 ml fassenden Rührautoklaven wurde eine Mischung aus 150 g Diphenylamin und 6 g Katalysator der Zusammensetzung 10 Gew.% Palladium und 5 Gew.% Praseodymoxid auf Aluminiumoxid bei 230°C und 200 bar Wasserstoffdruck bis zur Druckkonstanz hydriert. Das Reaktionsprodukt bestand aus reinem Dicyclohexylamin (Kp = 132 bis 134°C/27 mbar).

Beispiel 6

1-Dimethylamino-4-methylcyclohexan

Man verfuhr analog Beispiel 5, verwendete aber einen Katalysator, der 10 Gew.% Palladium, 0,11 Gew.% Zink und 0,10 Gew.% Cadmium auf Aluminiumoxid enthielt. Ausgehend von 1-Dimethylamino-4-methylbenzol wurde 1-Dimethylamino-4-methylcyclohexan als cis-/trans-Isomerengemisch (Kp = 156 bis 160°C) in einer Ausbeute von 94 % der Theorie erhalten.

Beispiel 7

1-Dimethylamino-2-propylcyclohexan

Man verfuhr analog Beispiel 5, verwendete aber einen Katalysator, der 10 Gew.% Palladium, 5 Gew.% Silber und 1 Gew.% Mangan auf Aluminiumoxid enthielt. Ausgehend von 1-Dimethylamino-2-propylbenzol wurde 1-Dimethylamino-2-propylcyclohexan als cis-/trans-Isomerengemisch (Kp = 206 bis 215°C) in einer Ausbeute von 94 % der Theorie erhalten.

Beispiel 8

1-Isopropylamino-2,6-dimethylcyclohexan

Man verfuhr analog Beispiel 5, verwendete aber als Ausgangsprodukt 1-Isopropylamino-2,6-dimethylbenzol und hydrierte bei 210°C bis zur Druckkonstanz. Als Reaktionsprodukt erhielt man 1-Isopropylamino-2,6-dimethylcyclohexan als cis/trans-Isomerengemisch (Kp = 86-87°C/20 mbar) in einer Ausbeute von 93 % d.Th.

Beispiel 9

1-Ethylamino-2-methyl-6-tert.-butylcyclohexan

Man verfuhr analog Beispiel 5, verwendete aber als Ausgangsprodukt 1-Ethylamino-2-methyl-6-tert.-butylbenzol und hydrierte bei 210°C bis zur Druckkonstanz. Als Reaktionsprodukt erhielt man 1-Ethylamino-2-methyl-6-tert.-butylcyclohexan als cis/trans-Isomerengemisch (Kp = 132-134°C/20 mbar) in einer Ausbeute von 91 % d.Th.

Beispiel 10

1-Ethylamino-2,6-diisopropylcyclohexan

Man verfuhr analog Beispiel 5, verwendete aber als Ausgangsprodukt 1-Ethylamino-2,6-diisopropylbenzol und hydrierte bei 210°C bis zur Druckkonstanz. 1-Ethylamino-2,6-diisopropylcyclohexan wurde als cis/trans-Isomerengemisch (Kp = 164-167°C/20 mbar) in einer Ausbeute von 94 % d.Th. erhalten.

Patentansprüche

1. Verfahren zur Herstellung von cycloaliphatischen Aminen durch Hydrierung von aromatischen Aminen in Gegenwart eines Hydrierkatalysators, wobei man einen Trägerkatalysator verwendet, der als Träger Aluminiumoxid oder eine Aluminiumoxid enthaltende Verbindung, Palladium und eine basische Verbindung und/oder ein Element der Gruppe Ib, IIb, VIIb, Eisen, Kobalt oder Nickel enthält, dadurch gekennzeichnet, daß man als aromatische Amine N-Alkyl- bzw. N-Arylaniline einsetzt.

2. Verfahren gemäß Anspruch 1, dadurch gekennzeichnet, daß der Katalysator als basischen Stoff ein Oxid, Hydroxid oder Carbonat eines Alkalimetalls, Erdalkalimetalls oder eines Seltenerdmetalls enthält.

3. Verfahren gemäß Anspruch 2, dadurch gekennzeichnet, daß der Katalysator eine Magnesiumverbindung enthält.

4. Verfahren gemäß Anspruch 2, dadurch gekennzeichnet, daß der Katalysator eine Praseodym-/Neodym- oder Cerverbindung enthält.

0108323

Europäisches Patentamt

# EUROPÄISCHER RECHERCHENBERICHT

Nummer der Anmeldung

EP 83 11 0597

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | HOUBEN-WEYL: "Methoden der Organischen Chemie", Band XI/1: "Stickstoffverbindungen II", 4. Auflage, 1957, Seiten 680-681, Georg Thieme Verlag, Stuttgart, DE<br>* Seite 681, Zeilen 12-13 * | 1-4 | C 07 C 85/24 |
| D,Y | EP-A-0 053 818 (BASF AG)<br>* Insgesamt * | 1-4 | |
| A | FR-A-1 343 391 (ABBOTT LABORATORIES)<br>* Zusammenfassung * | 1 | |
| D,A | US-A-3 376 341 (C.R. BAUER) | | |
| | | | RECHERCHIERTE SACHGEBIETE (Int. Cl. 3) |
| | | | C 07 C 85/00 |

Der vorliegende Recherchenbericht wurde für alle Patentansprüche erstellt.

| Recherchenort | Abschlußdatum der Recherche | Prüfer |
|---|---|---|
| DEN HAAG | 02-02-1984 | PAUWELS G.R.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82